(19) Europäisches Patentamt
European Patent Office
Office européen des brevets

(11) Veröffentlichungsnummer: **0 474 109 A1**

# (12) EUROPÄISCHE PATENTANMELDUNG

(21) Anmeldenummer: **91114438.4**

(51) Int. Cl.[5]: **C07D 235/28**, A61K 31/415

(22) Anmeldetag: **28.08.91**

(30) Priorität: **03.09.90 HU 574690**

(43) Veröffentlichungstag der Anmeldung:
**11.03.92 Patentblatt 92/11**

(84) Benannte Vertragsstaaten:
**AT BE CH DE DK ES FR GB GR IT LI LU NL SE**

(71) Anmelder: **RICHTER GEDEON VEGYESZETI GYAR R.T.**
**Gyömröi ut 19-21**
**H-1103 Budapest X(HU)**

(72) Erfinder: **Harsányi, Kálmán, Dr.**
**Fodor u. 12**
**H-1126 Budapest(HU)**
Erfinder: **Tétényi, Péter**
**Népstadion u. 9**
**H-1143 Budapest(HU)**
Erfinder: **Nagy, Tamás**
**Hunyadletj u. 28**
**H-1121 Budapest(HU)**
Erfinder: **Csehi, Attila**
**Lánchid u. 7**
**H-2131 Göd(HU)**
Erfinder: **Gizur, Tibor, Dr.**
**Kassai u. 161/b**
**H-1142 Budapest(HU)**
Erfinder: **Heged s, Béla, Dr.**
**Bart k B. u. 82**
**H-1113 Budapest(HU)**
Erfinder: **Maderspach, Andrea, Dr.**
**Vörösvári u. 13**
**H-1035 Budapest(HU)**
Erfinder: **Jávor, András**
**Torbágy u. 8**
**H-1118 Budapest(HU)**
Erfinder: **Haj s, György, Dr.**
**Gábor áron u. 59**
**H-1026 Budapest(HU)**
Erfinder: **Szporny, Lászl , dr.**
**Szabolcska M. u. 7**
**H-1114 Budapest(HU)**

(74) Vertreter: **Hansen, Bernd, Dr. Dipl.-Chem. et al**
**Hoffmann, Eitle & Partner Patent- und Rechtsanwälte Arabellastrasse 4 Postfach 81 04 20**
**W-8000 München 81(DE)**

## (54) Neue 5-Benzyl-substituierte Benzimidazolin-2-Thion-Derivate und Verfahren zu ihrer Herstellung.

(57) Die Erfindung betrifft antihyperlipoproteinämisch wirksame neue 5-benzyl-substituierte Benzimidazolin-2-thion-Derivate der allgemeinen Formel (I) und ein Verfahren zu ihrer Herstellung.

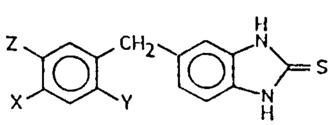

(I)

In der allgemeinen Formel (I) steht

- X für Halogen, Methyl-, Ethyl-, Methoxy- oder Ethoxygruppe,
- Y im Falle von X = Methoxy oder Ethoxygruppe für Hydroxylgruppe, anderenfalls für Wasserstoff, Methoxy- oder Ethoxygruppe und die Bedeutung von
- Z ist für den Fall, daß X oder Y für Methoxy- oder Ethoxygruppe beziehungsweise Wasserstoff steht, Methoxy- oder Ethoxygruppe, anderenfalls Wasserstoff.

EP 0 474 109 A1

Die Erfindung betrifft antihyperlipoproteinämisch wirksame neue 5-benzyl-substituierte Benzimidazolin-2-thion-Derivate der allgemeinen Formel (I) und ein Verfahren zu ihrer Herstellung.

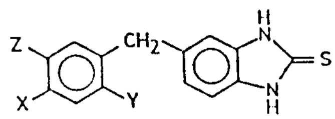

(I)

In der allgemeinen Formel (I) steht

X für Halogen, Methyl-, Ethyl-, Methoxy- oder Ethoxygruppe,

Y im Falle von X = Methoxy oder Ethoxygruppe für Hydroxylgruppe, anderenfalls für Wasserstoff, Methoxy- oder Ethoxygruppe und die Bedeutung von

Z ist für den Fall, daß X oder Y für Methoxy- oder Ethoxygruppe beziehungsweise Wasserstoff steht, Methoxy- oder Ethoxygruppe, anderenfalls Wasserstoff.

Die Verbindungen der allgemeinen Formel (I) können auch in den tautomeren Formen der allgemeinen Formeln (Ia) und (Ib) beschrieben werden.

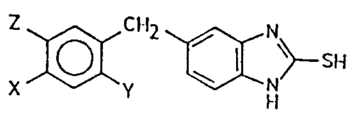

(I/a)

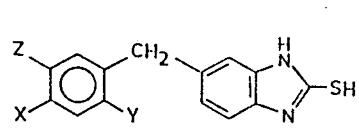

(I/b)

Der Einfachheit halber werden die Verbindungen jedoch im folgenden so betrachtet, als entspräche ihre Struktur derin Formel (I) dargestellten "Thion"-Form.

Zum Gegenstand der Erfindung gehören auch die die Verbindungen der allgemeinen Formel (I) enthaltenden, die Arterienverkalkung und Thrombosenbildung verhindernden, antihyperlipoproteinämisch wirksamen Arzneimittelpräparate, ihre Herstellung und ihre Verwendung in der Behandlung von Hyperlipoproteinämie.

Die Atherosklerose ist ein langsam progredierender Prozeß; eines seiner Hauptmerkmale ist, daß sich die Lipidkomponenten des Plasmas, zum Beispiel die Cholesterinester, in den Läsionen der Gefäßwand anreichern. Der Prozeß beginnt mit einer Verletzung der endothelen Schicht der Gefäßwand. An der Stelle der Verletzung haften die Blutplättchen an, aus ihnen werden unterschiedliche Stoffe freigesetzt, die die Zellen der glatten Muskulatur der Gefäßwand zur Vermehrung anregen.

1984 einigten sich die Fachleute dahingehend, daß der Grund für die koronaren Herzerkrankungen neben sonstigen Risikofaktoren wie hoher Blutdruck, Rauchen, Zuckerkrankheit usw. in erster Linie der hohe Cholesterinspiegel im Blutserum ist (Consensus Development Conference: JAMA 1985, 2080-2086). Da bei den meisten der Kranken der Anstieg des Cholesterinspiegels nicht allein auftritt, wurde vorgeschlagen (National Cholesterol Education Program Expert Panel on Detection, Evaluation and Treatment og High Blood Cholesterol in Adults: Arch. Intern. Med., 1988, 148, 36-39), den Cholesterinspiegel des Blutserums auf einem Wert von 200 mg/dl zu halten. Im Blut liegt das Cholesterin an Lipoproteine gebunden vor. Unter diesem Aspekt ist besonders die LDL-Fraktion (low density lipoprotein) wichtig, die 60-75 % des im Blut kreisenden Cholesterins trägt und die gefährlichste Komponente darstellt. Eine Senkung dieser Komponente ist daher besonders wünschenswert. Beim LDL-Cholesterinspiegel werden abhängend von den übrigen Risikofaktoren Werte von 130-160 mg/dl angestrebt. Das sind derart strenge Bedingungen, daß sie nur mit Arzneimitteln erreicht werden können. Aus diesem Grund ist der Bedarf an den Cholesterinspiegel senkenden Arzneimitteln angestiegen. Da das Ziel aber nicht nur in der Senkung des Gesamtcholesterinspiegels im Serum, sondern darüber hinaus auch in einer vorteilhaften Änderung des Verhältnisses der das Cholesterin tragenden Proteinfraktionen liegt, werden nicht nur die neuen, modernen, die Biosynthese des Cholesterins hemmenden Arzneimittel gebraucht, sondern auch Wirkstoffe, die neben einer Verminderung des Gesamtcholesterins und des LDL-Cholesterins auch eine Erhöhung der eine Schutzwirkung ausübenden HDL-Fraktion (high density fraction) bewirkt. Auf Grund neuerer Forschungsergebnisse scheint es wünschenswert, wenn auch der als unabhängiger Risikofaktor betrachtete Triglyceridspiegel gesenkt wird.

Zur Senkung des Cholesterin- und Triglyceridspiegels haben bestimmte Aryloxyalkancarbonsäuren

Eingang in die therapeutische Praxis gefunden, von denen das Clofibrat, d.h. der 2-(4-Chlorphenoxy)-2-methyl-propionsäureethylester, als Pionierverbindung betrachtet werden kann. Auch Verbindungen ähnlicher Struktur gelangten in den Handel. Diese Struktur steht den erfindungsgemäßen Verbindungen jedoch sehr fern.

Verbindungen ähnlicher Struktur wie die gemäß der vorliegenden Erfindung sind aus der ungarischen Patentschrift Nr. 193 951 bekannt. Die dort beschriebenen schwefelhaltigen Benzimidazole unterscheiden sich von den erfindungsgemäßen Verbindungen dadurch, daß sie in 2-Stellung einen S-Alkyl-Substituenten tragen, während die erfindungsgemäßen Verbindungen dort ein Schwefelatom =S aufweisen. Ferner tragen die erfindungsgemäßen Verbindungen in 5-Stellung als Substituenten eine im Ring immer substituierte Benzylgruppe. Infolge dieser Unterschiede ist auch die Wirkung der neuen Verbindungen stärker, wie die Ergebnisse der pharmakologischen Untersuchungen zeigen.

Erfindungsgemäß werden die Verbindungen der allgemeinen Formel (I) hergestellt, indem man in 4-Stellung substituierte 1,2-Diaminobenzole der allgemeinen Formel (II)

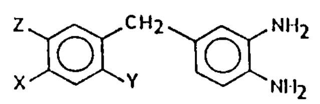

(II)

worin die Bedeutung von X, Y und Z die gleiche wie oben ist mit einem Thiokohlensäurederivat der allgemeinen Formel (III)

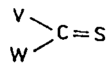

(III)

worin

| | |
|---|---|
| V und W | unabhängig voneinander für Chloratom oder Aminogruppe stehen, oder |
| V | eine Gruppe der allgemeinen Formel Me-S- ist, worin Me für Alkalimetall steht, und W Methoxy- oder Ethoxygruppe bedeutet, oder |
| V und W | zusammen für ein weiteres Schwefelatom oder einzeln für je eine Imidazolylgruppe stehen, |

umsetzt.

Es ist besonders vorteilhaft, die Verbindungen der allgemeinen Formel (II) mit Verbindungen der allgemeinen Formel (III) umzusetzen, in denen V eine Gruppe der allgemeinen Formel Me-S- und W Methoxy- oder Ethoxygruppe bedeutet. Eine solche Verbindung ist zum Beispiel das Kaliumethylxanthogenat, das eine feste, stabile Substanz dasstellt. Die Synthese wird gemäß Org. Synth. Coll. Vol. 4, 569 (1963) vorgenommen. Die Ausgangsstoffe der allgemeinen Formel (II) können aus statt der $-CH_2$-Gruppe eine -CO-Gruppe enthaltenden Verbindungen durch Reduktion hergestellt werden. Die Synthese des 3,4-Diaminobenzophenons ist aus der Literatur bekannt, wenn auch die als X, Y und Z die angegebenen Substituenten enthaltenden Diaminobenzophenone neue Verbindungen sind.

Zum Erfindungsgegenstand gehören weiterhin die als Wirkstoffe Verbindungen der allgemeinen Formel (I) enthaltenden Arzneimittelpräparate sowie auch ein Verfahren zu ihrer Herstellung. Zur Herstellung der Arzneimittelpräparate werden eine oder mehrere Verbindungen der allgemeinen Formel (I) mit den in der Herstellung von Arzneimitteln üblichen Füll-, Streck- und Verdünnungsstoffen, Stabilisatoren, Geschmacks- und Aromastoffen, Lösungsmitteln, Netzmitteln, oberflächenaktiven Stoffen und Formulierungshilfen vermischt und zu je Dosiereinheit vorzugsweise 20-500 mg Wirkstoff enthaltenden Darreichungsformen formuliert.

Die Wirksamkeit der erfindungsgemäßen Verbindungen wurde auf die folgende Weise untersucht.

Untersuchungsmethoden

140-160 g schwere Hannover-Wistar-Ratten wurden 7 Tage lang mit 1,5 % Cholesterin und 0,5 % Natriumcholat enthaltendem Rattenfutter "LATI" [Schurr, P. E., Schultz, J. R., Day, C. E.: Atherosclerosis Drug Discovery, Ed: C. E. Day; Plenum Press, New York, 215 (1976)] gefüttert. Durch die Wirkung des Futters stieg der Cholesterinspiegel der Tiere um 200-250 % an, die Menge des HDL-Cholesterins ging um 50 % zurück. Die Tiere wurden in Sechsergruppen eingeteilt. Die Behandlung mit den Verbindungen wurde

am 4. Tag der Verabreichung des cholesterinhaltigen Futters begonnen und bis zum Ende des Versuches fortgesetzt. Die Verbindungen wurden in Form einer Suspension per os verabreicht. Nach Ablauf der Behandlungszeit ließ man die Tiere 18 Stunden lang hungern und danach in Äthernarkose ausbluten. Im Blutserum wurden der Gesamtcholesteringehalt, das HDL-Cholesterin und die Triglyceride mittels der Enzymteste von Beckman bestimmt. LDL + VLDL wurden mit Heparin-Mangan ausgefällt und turbidimetrisch bestimmt [Schurr, P. E., Schultz, J. R., Day, C. E.: Atherosclerosis Drug Discovery, Ed: C. E. Day; Plenum Press, New York, 215 (1976)].

Mit der am besten wirksamen Verbindung, der Verbindung gemäß Beispiel 3 (im folgenden: RGH-4819) wurden auch Untersuchungen zum Wirkungsmechanismus vorgenommen. Zu den Untersuchungen wurden mittels Triton WR-1339 (Polymer aus Octylphenolpolyethylenglycolether und Formaldehyd, Hersteller: Serva Feinbiochemica GmbH et Co., Heidelberg, BRD) hyperlipidämisch gemachte Ratten herangezogen. Die Tiere wurden 10 Tage lang per os mit entsprechenden Dosen der Verbindungen behandelt und bekamen 6 Stunden vor ihrer Tötung das Triton als einmalige Dosis in die Vene. Durch Triton WR-1339 steigt der Cholesterinspiegel des Blutserums auf das 1,5-fache seines Wertes, und der Triglyceridspiegel steigt von dem Normalwert von 60-70 mg/dl auf 800-1500 mg/dl. Der Cholesterinspiegel steigt in dem sechsstündigen Versuch infolge einer Intensivierung der Biosynthese. In dieser Zeitspanne kann indirekt auf eine Hemmung der Cholesterin-Biosynthese geschlossen werden. Die hohen Triglyceridwerte haben ihren Grund in einer Hemmung des Abbauprozesses, indem das Triton WR-1339 als oberflächenaktive Substanz die Funktion des am Katabolismus der Triglyceride beteiligten Enzyms Lipoproteinlipase hemmt.

Als Referenzsubstanz wurde in den Untersuchungen das aus der ungarischen Patentschrift Nr. 193 951 bekannte 5-Benzyl-2-(2-propenylthio)-benzimidazol-hydrochlorid (im folgenden:0202479) verwendet, das den neuen Verbindungen chemisch nahesteht.

Die Ergebnisse sind in den Tabellen 1, 2 und 3 zusammengefaßt. In der Tabelle 1 sind die Ergebnisse der Untersuchungen an mit cholesterinhaltigem Futter gefütterten Ratten enthalten. Die Tabelle 2 zeigt den Dosis-Wirkung-Zusammenhang in einem 10-Tageversuch (mit Cholesterin gefütterte Ratten). In der Tabelle 3 sind die experimentellen Daten hinsichtlich des Wirkungsmechanismus aus dem oben beschriebenen Versuch angegeben.

In den kurzzeitigen Untersuchungen (7 Tage cholesterinhaltiges Futter, 4 Tage Behandlung) zeigten die erfindungsgemäßen Verbindungen eine ausgesprochen cholesterinspiegelsenkende Wirkung. Sie senkten auch den Triglycerid- und LDL+VLDL-Spiegel bedeutend. In der Änderung des Spiegels des eine Schutzwirkung ausübenden HDL zeigten die Verbindungen eine unterschiedliche Wirkung (Tabelle 1).

Die Ergebnisse der auf mehreren Dosisebenen durchgeführten Untersuchungen zum Dosis-Wirkung-Zusammenhang (Tabelle 1) zeigen, daß die erfindungsgemäße Verbindung den Cholesterinspiegel des Blutes proportional zur Dosis senkte. Ähnlich gute Ergebnisse waren im Falle der aterogen wirkenden LDL-VLDL-Fraktion zu verzeichnen. Auch die Menge der schützend wirkenden HDL-Fraktion stieg etwas an. Die Senkung des Triglyceridspiegels war mäßiger. Diese Wirkungen übertrafen die Wirkung der Referenzsubstanz.

Die Ergebnisse der an mit Triton WR-1339 hyperlipidämisch gemachten Ratten durchgeführten Untersuchungen zum Wirkungsmechanismus zeigen, daß die erfindungsgemäße Verbindung wirksamer ist als die Referenzsubstanz. Die grössere Wirkung zeigt sich in erster Linie in einer Senkung des Triglyceridspiegels, aber auch die Senkung des Cholesterinspiegels ist dosisabhängig. Die Daten lassen darauf schliesen, daß der Wirkstoff entweder die Biosynthese oder die Resorption des Cholesterins hemmt. Die Senkung des Triglyceridspiegels ist sicher darauf zurückzuführen, daß der Wirkstoff das im Abbau der Triglyceride eine wichtige Rolle spielende, durch das Triton gehemmte Enzym Lipoproteinlipase aktiviert.

Tabelle 1

Lipidsenkende Aktivitäten an mit Cholesterin gefütterten Ratten (7 Tage Cholesterinfütterung, 4 Tage Behandlung)

| Verbindung | Dosis | Serum (Änderung in %) | | | |
|---|---|---|---|---|---|
| | mg/kg p.o. | Chole-sterin | Trigly-cerid | LDL+ VLDL | HDL-Cho-lesterin |
| Beispiel 2 | 30,0 | -22,3 | -22,9 | -35,4 | 40,6 |
| Beispiel 3 | 30,0 | -39,7 | -35,7 | -73,6 | 15,1 |
| Beispiel 4 | 30,0 | -34,6 | 0,0 | -45,3 | 64,3 |
| Beispiel 1 | 30,0 | -26,1 | 0,0 | -38,3 | -20,8 |
| Beispiel 7 | 30,0 | -36,2 | -64,5 | -36,1 | 16,7 |
| Beispiel 5 | 30,0 | -43,5 | -43,2 | -53,5 | 2,0 |
| Beispiel 6 | 30,0 | -33,0 | -33,0 | -33,9 | -7,6 |
| 0202479 | 30,0 | -36,5 | -22,3 | -50,2 | -24,6 |

Tabelle 2

Wirkung von RGH-4819 an mit Cholesterin gefütterten Ratten (10-tägiger Versuch)

| Verbindung | Dosis mg/kg p.o. | Serum (Änderung in %) Chole-sterin | Trigly-cerid | LDL+ VLDL | HDL-Cho-lesterin |
|---|---|---|---|---|---|
| Beispiel 3 | 1,0 | -4,4 | 23,5 | -21,7 | 4,9 |
| Beispiel 3 | 3,0 | -33,1** | -14,8 | -45,8** | 0,5 |
| Beispiel 3 | 10,0 | -53,8** | -28,5* | -72,7** | 18,6* |
| Beispiel 3 | 30,0 | -46,5** | -18,4 | -68,7** | 16,6 |
| 0202479 | 3,0 | -3,6 | -1,4 | -15,9 | 9,4 |
| 0202479 | 10,0 | -13,2 | -13,0 | -28,1* | 10,5 |
| 0202479 | 30,0 | -36,5** | -22,3 | -50,2** | 24,6* |

Tabelle 3

Wirkung von RGH-4819 an mit Triton WR-1339 behandelten Ratten; 6stündige Tritonlipämie

| Verbindung | Dosis mg/kg p.o. | Serum (Änderung in %) Cholesterin | Triglycerid |
|---|---|---|---|
| Beispiel 3 | 10,0 | -19,4 | -36,0** |
| Beispiel 3 | 30,0 | -24,9 | -40,7** |
| Beispiel 3 | 100,0 | -28,7 | -48,5** |
| 0202479 | 30,0 | -20,4 | -19,1 |
| 0202479 | 100,0 | -25,2 | -18,2 |

* = 0,01 < P < 0,05; ** = P < 0,01

Weitere Einzelheiten der Erfindung werden in den folgenden Beispielen beschrieben, die jedoch keinen ein-schränkenden Charakter haben.

**Beispiel 1**

*5-[(4-Chlorphenyl)-methyl]-2,3-dihydro-1H-benzimidazol-2-thion*

6,54 g (99 mMol) 85 %-iges festes Kaliumhydroxyd werden in einem Gemisch aus 40 ml Ethanol und 18 ml Wasser gelöst und 13,75 g (45 mMol) 4-[(4-Chlorphenyl)-methyl]-o-phenylendiamin-dihydrochlorid zu der Lösung gegeben. Das Gemisch wird bei 60 °C so lange gerührt, bis sich alles gelöst hat (etwa 5 Minuten). Danach wird die Lösung mit 8,65 g (54 mMol) O-Ethyl-S-kaliumdithiocarbonat versetzt und 9 Stunden lang gekocht. Dann wird das Reaktionsgemisch auf 400 ml Wasser gegossen und unter ständigem Rühren mit kleinen Portionen Essigsäure (etwa 10 ml) angesäuert. Die Suspension wird eine weitere Stunde

lang gerührt, dann wird das Produkt abfiltriert und mit 5x80 ml Wasser sulfidfrei gewaschen. Das nutschfeuchte Produkt wird in kochendem n-Butanol gelöst und die Lösung mit Aktivkohle geklärt. Das kristallisierte Produkt schmilzt bei 275-280 °C. Ausbeute: 10,3 g (83 %).

**Beispiel 2**

*5-[4-Methylphenyl)-methyl]-2,3-dihydro-1H-benzimidazol-2-thion*

Die Titelverbindung wird auf die im Beispiel 1 beschriebene Weise aus 12,83 g (45 mMol) 4-[(4-Methylphenyl)-methyl]-o-phenylendiamin-dihydrochlorid hergestellt. Das Rohprodukt wird aus n-Butanol umkristallisiert. Ausbeute: 9,77 g (85 %), Schmp.: 258-260 °C

**Beispiel 3**

*5-[4-Methoxyphenyl)-methyl]-2,3-dihydro-1H-benzimidazol-2-thion*

1,15 g (17 mMol) 85 %iges festes Kaliumhydroxyd werden in einem Gemisch aus 70 ml Ethanol und 23 ml Wasser gelöst. Zu der Lösung werden 19 g (83,2 mMol) 4-[(4-Methoxyphenyl)-methyl]-o-phenylendiam in-dihydrochlorid und 15,4 g (95,8 mMol) O-Ethyl-S-kaliumdithiocarbonat gegeben. Das Gemisch wird 9 Stunden lang gekocht und dann auf 900 ml Wasser gegossen. Das rohe Produkt wird auf die im Beispiel 1 beschriebene Weise isoliert und im nutschfeuchten Zustand in 800 ml Isopropanol gelöst. Nach in der Hitze vorgenommenem Klären mit Aktivkohle wird die Verbindung kristallisiert. Ausbeute: 18,0 g (80 %), Schmp.: 254-255 °C.

**Beispiel 4**

*5-[4-Methoxyphenyl)-methyl]-2,3-dihydro-1H-benzimidazol-2-thion*

Zu einem Gemisch aus 22 ml Ethanol, 3,5 ml Wasser und 0,83 g (12,6 mMol) 85 %igem Kaliumhydroxyd werden 2,5 g (10,95 mMol) 4-[(4-Methoxyphenyl)-methyl]-o-phenylendiamin und 0,96 g (0,76 ml, 12,6 mMol) Schwefelkohlenstoff gegeben. Das Gemisch wird 5 Stunden lang am Rückfluß gekocht, dann abgekühlt, mit 25 ml Wasser versetzt und dann unter Eiskühlung von außen und intensivem Rühren mit 2,80 g 50 %iger Essigsäure (23,3 mMol) angesäuert. Nach einstündigem Rühren wird das Produkt abfiltriert und mit 3x20 ml Wasser gewaschen. Das nutschfeuchte Produkt wird in 70 ml Isopropanol gelöst und nach in der Hitze vorgenommenem Klären mit Aktivkohle kristallisiert. Ausbeute: 1,64 g (55,4 %) der weißen kristallinen Titelverbindung. Schmp.: 254-255 °C.

**Beispiel 5**

*5-[4-Methoxyphenyl)-methyl]-2,3-dihydro-1H-benzimidazol-2-thion*

Ein Gemisch aus 0,35 g (1,16 mMol) 4-[(4-Methoxyphenyl)-methyl]-o-phenylendiamin-dihydrochlorid und 0,286 g (3,76 mMol) Thioharnstoff wird auf dem Ölbad bei 150-169 °C geschmolzen und zwei Stunden lang bei dieser Temperatur gehalten. Nach dem Abkühlen wird das Gemisch mit 10 %iger wässriger Natriumcarbonatlösung verrieben und die erhaltene Suspension eine Stunde lang gerührt. Das Rohprodukt wird abfiltriert, viermal mit Wasser gewaschen und dann aus Isopropanol nach in der Hitze mit Aktivkohle vorgenommenem Klären kristallisiert. Ausbeute: 88 mg (28 %), Schmp.: 252-254 °C.

**Beispiel 6**

*5-[4-Methoxyphenyl)-methyl]-2,3-dihydro-1H-benzimidazol-2-thion*

Zu der Lösung von 1,14 g (5,0 mMol) 4-[(4-Methoxyphenyl)-methyl]-o-phenylendiamin in 30 ml wasserfreiem Tetrahydrofuran wird unter Außenkühlung mit Wasser tropfenweise die Lösung von 0,98 g (5,5 mMol) N,N'-Thiocarbonylimidazol in 15 ml wasserfreiem Tetrahydrofuran gegeben. Das Reaktionsgemisch wird bei Raumtemperatur 5 Stunden lang gerührt, dann mit 1,5 ml Wasser versetzt und eine weitere halbe Stunde lang gerührt. Dann wird das Lösungsmittel im Vakuum abgezogen und der Eindampfrückstand mit Wasser behandelt. Das Rohprodukt wird abfiltriert, dreimal mit Wasser gewaschen und auf die im Beispiel 5

beschriebene Weise aus Isopropanol umkristallisiert. Ausbeute: 0,9 g (51,0 %), Schmp.: 253-255 °C.

**Beispiel 7**

*5-[4-Methoxyphenyl)-methyl]-2,3-dihydro-1H-benzimidazol-2-thion*

Zu der Lösung von 0,51 g (5,0 mMol) Triethylamin und 1,14 g (5,0 mMol) 4-[(4-Methoxy-phenyl)-methyl]-o-phenylendiamin in 35 ml Chloroform wird unter Außenkühlung mit Wasser tropfenweise die Lösung von 0,58 g (5,0 mMol) Thiophosgen in 10 ml Chloroform gegeben. Das Reaktionsgemisch wird bei Raumtemperatur eine Stunde lang gerührt, dann wird das Lösungsmittel im Vakuum entfernt. Der Eindampfrückstand wird mit 5 %iger Natriumcarbonatlösung verrieben und nach zweistündigem Rühren abfiltriert. Das Rohprodukt wird dreimal mit Wasser gewaschen und dann auf die im Beispiel 5 beschriebene Weise aus Isopropanol umkristallisiert. Ausbeute: 0,68 g (50,3 %), Schmp.: 254-255 °C.

**Beispiel 8**

*5-[3,4-Dimethoxyphenyl)-methyl]-2,3-dihydro-1H-benzimidazol-2-thion*

Die Titelverbindung wird aus 18,30 g (70,9 mMol) 4-[(3,4-Dimethoxyphenyl)-methyl]-o-phenylendiamin unter Einhaltung der im Beispiel 3 angegebenen Molverhältnisse und Reaktionsbedingungen hergestellt. Das Rohprodukt wird durch Umkristallisieren aus n-Butanol gereinigt. Ausbeute: 7,1 g (33 %), Schmp.: 253-256 °C.

**Beispiel 9**

*5-[2-Hydroxy-4-methoxyphenyl)-methyl]-2,3-dihydro-1H-benzimidazol-2-thion*

Die Titelverbindung wird aus 22,83 g (50,0 mMol) 4-[(2-Hydroxy-4-methoxyphenyl)-methyl]-o-phenylendiamin unter Einhaltung der im Beispiel 3 angegebenen Molverhältnisse und Reaktionsbedingungen hergestellt. Das Rohprodukt wird durch Umkristallisieren aus n-Butanol gereinigt. Ausbeute: 14,0 g (49 %), Schmp.: 248-250 °C.

**Beispiel 10**

*5-[4-Ethoxyphenyl)-methyl]-2,3-dihydro-1H-benzimidazol-2-thion*

Die Titelverbindung wird aus 12,1 g (50,0 mMol) 4-[(4-Ethoxyphenyl)-methyl]-o-phenylendiamin unter Einhaltung der im Beispiel 3 angegebenen Molverhältnisse und Reaktionsbedingungen hergestellt. Das Rohprodukt wird durch Umkristallisieren aus n-Butanol gereinigt. Ausbeute: 11,6 g (82 %), Schmp.: 243-246 °C.

**Beispiel 11**

*5-[2,5-Dimethoxyphenyl)-methyl]-2,3-dihydro-1H-benzimidazol-2-thion*

Die Titelverbindung wird aus 18,30 g (70,9 mMol) 4-[(2,5-Dimethoxyphenyl)-methyl]-o-phenylendiamin unter Einhaltung der im Beispiel 3 angegebenen Molverhältnisse und Reaktionsbedingungen hergestellt. Das Rohprodukt wird durch Umkristallisieren aus n-Butanol gereinigt. Ausbeute: 15,5 g (73 %), Schmp.: 210-211 °C.

**Beispiel 12**

*Herstellung von Tabletten mit 30 g Wirkstoff*

Mittels der üblichen Methoden der Tablettenbereitung werden Tabletten von 9 mm Durchmesser und 250 mg Masse gemäß der folgenden Zusammensetzung hergestellt:

| | |
|---|---|
| 5-[4-Methoxyphenyl)-methyl]-2,3-dihydro-1H-benzimidazol-2-thion | 30,0 mg |
| Lactose | 130,0 mg |
| Stärke | 64,0 mg |
| Polyvinylpyrrolidon (Polyvidon) | 5,0 mg |
| mikrokristalline Cellulose | 10,0 mg |
| Talkum | 7,5 mg |
| Magnesiumstearat | 2,5 mg |
| kolloide Kieselsäure | 1,0 mg |

**Beispiel 13**

*Herstellung von 125 mg Wirkstoff enthaltenden Kapseln*

Selbstschließende Hartgelatinekapseln der Größe 1 werden mit einem Gemisch der folgenden Zusammensetzung gefüllt:

| | |
|---|---|
| 5-[4-Methoxyphenyl)-methyl]-2,3-dihydro-1H-benzimidazol-2-thion | 150,0 mg |
| Polyvinylpyrrolidon (Polyvidon) | 5,0 mg |
| mikrokristalline Cellulose | 58,0 mg |
| Talkum | 6,0 mg |
| Magnesiumstearat | 5,0 mg |
| kolloide Kieselsäure | 1,0 mg |

**Patentansprüche**

1. Verfahren zur Herstellung von antihyperlipoproteinämisch wirksamen neuen 5-benzylsubstituierten Benzimidazolin-2-thion-Derivaten der allgemeinen Formel (I)

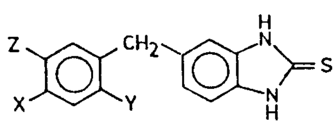

worin
X für Halogen, Methyl-, Ethyl-, Methoxy- oder Ethoxygruppe steht,
Y im Falle von X = Methoxy oder Ethoxygruppe Hydroxylgruppe, anderenfalls Wasserstoff, Methoxy- oder Ethoxygruppe bedeutet und die Bedeutung von
Z für den Fall, daß X oder Y für Methoxy- oder Ethoxygruppe beziehungsweise Wasserstoff steht, Methoxy- oder Ethoxygruppe, anderenfalls Wasserstoff ist,
dadurch **gekennzeichnet,** daß man
in 4-Stellung substituierte 1,2-Diaminobenzole der allgemeinen Formel (II)

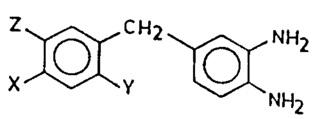

(II)

worin die Bedeutung von X, Y und Z die gleiche wie oben ist, mit einem Thiokohlensäurederivat der allgemeinen Formel (III)

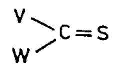

(III)

worin

V und W unabhängig voneinander für Chloratom oder Aminogruppe stehen, oder

V eine Gruppe der allgemeinen Formel Me-S- ist, worin Me für Alkalimetall steht, und W Methoxy- oder Ethoxygruppe bedeutet, oder

V und W zusammen für ein weiteres Schwefelatom oder einzeln für je eine Imidazolylgruppe stehen,

umsetzt.

**2.** Verfahren nach Anspruch 1, dadurch gekennzeichnet, daß man Verbindungen der allgemeinen Formel (III) einsetzt, in denen V eine Gruppe der allgemeinen Formel Me-S- mit Me = Kalium- oder Natriumatom bedeutet und W für Methoxy- oder Ethoxygruppe steht.

**3.** Verfahren nach Anspruch 1, dadurch gekennzeichnet, daß man Verbindungen der allgemeinen Formel (III) einsetzt, in denen V und W zusammen für ein Schwefelatom stehen.

**4.** Verfahren nach Anspruch 1, dadurch gekennzeichnet, daß man Verbindungen der allgemeinen Formel (III) einsetzt, in denen V und W für je eine Aminogruppe stehen.

**5.** Verfahren nach Anspruch 1, dadurch gekennzeichnet, daß man Verbindungen der allgemeinen Formel (III) einsetzt, in denen V und W für je ein Chloratom stehen.

**6.** Verfahren nach Anspruch 1, dadurch gekennzeichnet, daß man Verbindungen der allgemeinen Formel (III) einsetzt, in denen V und W für je eine 1-Imidazolylgruppe stehen.

**7.** Verfahren zur Herstellung von Arzneimitteln mit antihyperlipoproteinämischer Wirkung, dadurch gekennzeichnet, daß man Verbindungen der allgemeinen Formel (I), worin die Bedeutung von X, Y und Z die gleiche wie im Anspruch 1 ist, mit den in der Arzneimittelindustrie üblichen Streck-, Träger- und Hilfsstoffen, Stabilisatoren, Zusätzen zur Beeinflussung des pH, des osmotischen Druckes, Geruchs- und Geschmacksstoffen, Formulierungshilfen etc. vermischt und zu Arzneimitteln formuliert.

**8.** Neue 5-benzyl-substituierte, antihyperlipoproteinämisch wirkende Benzimidazolin-2-thion-Derivate der allgemeinen Formel (I)

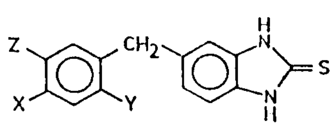

worin

X für Halogen, Methyl-, Ethyl-, Methoxy- oder Ethoxygruppe steht,

Y im Falle von X = Methoxy oder Ethoxygruppe Hydroxylgruppe, anderenfalls Wasserstoff, Methoxy- oder Ethoxygruppe bedeutet und die Bedeutung von

Z für den Fall, daß X oder Y für Methoxy- oder Ethoxygruppe beziehungsweise Wasserstoff steht, Methoxy- oder Ethoxygruppe, anderenfalls Wasserstoff ist,

**9.** 5-[(4-Chlorphenyl)-methyl]-2,3-dihydro-1H-benzimidazol-2-thion.

**10.** 5-[(4-Methylphenyl)-methyl]-2,3-dihydro-1H-benzimidazol-2-thion.

**11.** 5-[(4-Methoxylphenyl)-methyl]-2,3-dihydro-1H-benzimidazol-2-thion.

**12.** 5-[(3,4-Dimethoxylphenyl)-methyl]-2,3-dihydro-1H-benzimidazol-2-thion.

**13.** 5-[(2-Hydroxy-4-methoxylphenyl)-methyl]-2,3-dihydro-1H-benzimidazol-2-thion.

**14.** 5-[(4-Ethoxylphenyl)-methyl]-2,3-dihydro-1H-benzimidazol-2-thion.

**15.** 5-[(2,5-Dimethoxylphenyl)-methyl]-2,3-dihydro-1H-benzimidazol-2-thion.

**16.** Antihyperlipoproteinämisch wirksame Arzneimittelpräparate, dadurch gekennzeichnet, daß sie als Wirkstoff eine therapeutisch wirksame Menge einer Verbindung der allgemeinen Formel (I), worin die Bedeutung von X, Y und Z die gleiche wie im Anspruch 1 ist, zusammen mit den in der Arzneimittelindustrie üblichen Streck-, Träger- und Hilfsstoffen, Stabilisatoren, Zusätzen zur Beeinflussung des pH, des osmotischen Druckes, Geruchs- und Geschmacksstoffen, Formulierungshilfen etc. enthalten.

**17.** Behandlungsverfahren gegen Hyperlipoproteinämie, dadurch gekennzeichnet, daß man dem zu behandelnden Säuger, eingeschlossen auch den Menschen, eine therapeutisch wirksame Dosis eines 5-benzyl-substituierten Benzimidazolin-2-thion-Derivates der allgemeinen Formel (I), worin die Bedeutung von X, Y und Z die gleiche wie im Anspruch 1 ist, an sich oder in Form eines Arzneimittels verabreicht.

Europäisches Patentamt

**EUROPÄISCHER TEILRECHERCHENBERICHT,** der nach Regel 45 des Europäischen Patentübereinkommens für das weitere Verfahren als europäischer Recherchenbericht gilt

Nummer der Anmeldung: EP 91114438.4

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | CH - A5 - 634 307 (CIBA GEIGY AG) * Zusammenfassung * -- | 1,2,7, 8,16 | C 07 D 235/28<br>A 61 K 31/415 |
| A | EP - A1 - 0 190 817 (SMITHKLINE BECKMANN CORPORATION) * Zusammenfassung * ---- | 1,2,7, 8,16 | |

RECHERCHIERTE SACHGEBIETE (Int. Cl.5)

C 07 D 235/00

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-16

Unvollständig recherchierte Patentansprüche: -

Nicht recherchierte Patentansprüche: 17 (Verfahren zur thera-

Grund für die Beschränkung der Recherche:

peutischen Behandlung des menschlichen oder tierischen Körpers, Art. 52(4) EPÜ)

| Recherchenort | Abschlußdatum der Recherche | Prüfer |
|---|---|---|
| WIEN | 18-10-1991 | BRUS |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1505.1 03.82